(19) Europäisches Patentamt
European Patent Office
Office européen des brevets

(11) EP 1 666 501 A1

(12) **EUROPEAN PATENT APPLICATION**
published in accordance with Art. 158(3) EPC

(43) Date of publication:
**07.06.2006 Bulletin 2006/23**

(21) Application number: **04771773.1**

(22) Date of filing: **11.08.2004**

(51) Int Cl.:
***C07K 16/30*** *(1995.01)* ***C12N 15/12*** *(1990.01)*
***C12P 21/08*** *(1990.01)*

(86) International application number:
**PCT/JP2004/011812**

(87) International publication number:
**WO 2005/014651 (17.02.2005 Gazette 2005/07)**

(84) Designated Contracting States:
**AT BE BG CH CY CZ DE DK EE ES FI FR GB GR HU IE IT LI LU MC NL PL PT RO SE SI SK TR**

(30) Priority: **11.08.2003 JP 2003207165**

(71) Applicant: **CHUGAI SEIYAKU KABUSHIKI KAISHA**
**Tokyo, 115-8543 (JP)**

(72) Inventors:
- **TSUCHIYA, Masayuki,**
**c/o CHUGAI SEIYAKU K.K.**
**Gotenba-shi,**
**Shizuoka 4128513 (JP)**
- **IIJIMA, Shigeyuki,**
**c/o CHUGAI SEIYAKU K.K.**
**Gotenba-shi,**
**Shizuoka 4128513 (JP)**
- **SUGO, Izumi,**
**c/o CHUGAI SEIYAKU K.K.**
**Gotenba-shi,**
**Shizuoka 4128513 (JP)**
- **SUGIMOTO, Masamichi,**
**c/o CHUGAI SEIYAKU K.K.**
**Kamakura-shi,**
**Kanagawa 2478530 (JP)**

(74) Representative: **Harding, Charles Thomas**
**D Young & Co**
**120 Holborn**
**London EC1N 2DY (GB)**

(54) **SUGAR CHAIN-MODIFIED ANTI-HM1.24 ANTIBODY**

(57) An antibody (anti-HM1.24 antibody) against HM1.24 antigen in which the alteration of sugar chains resulted in enhanced antibody-dependent cellular cytotoxicity (ADCC), specifically an antibody having a sugar chain that does not contain α-1,6 core fucose, an antibody having a sugar chain that contains a bisecting N-acetylglucosamine (GlcNAc) structure, or an antibody having a sugar chain that does not contain α-1,6 core fucose and having a sugar chain that contains a bisecting N-acetylglucosamine (GlcNAc) structure.

EP 1 666 501 A1

## Description

FIELD OF THE INVENTION

The present invention relates to an antibody (anti-HM1.24 antibody) against HM1.24 antigen, said antibody having an enhanced antibody-dependent cellular cytotoxicity (ADCC), and a method of producing said antibody.

BACKGROUND ART

HM1.24 antigen is a membrane protein with a molecular weight of 29-33 kDa that is strongly expressed on the surface of myeloma cells (Ishikawa J. et al., Genomics 26 (1995), 527-534). The expression has also been noted in B tumor cells and T tumor cells, in addition to myeloma cells. In normal cells, the expression has been confirmed in immunoglobulin-producing B cells and activated T cells but little expression has been noted in other cells (Goto T. et al., Blood 84 (1994), 1922-1930).

As antibody (anti-HM1.24 antibody) against HM1.24 antigen is specifically accumulated in tumors due to the above tissue distribution of HM1.24 antigen, the antibody is very promising in applications such as the diagnosis of tumor localization and missile therapies such as radioimmunotherapy by radiolabelling the antibody and, as the antibody per se has antibody-dependent cellular cytotoxicity (ADCC) (Ozaki S. et al., Blood 90 (1997), 3179-3186), its use as a therapeutic agent for a myeloma such as multiple myeloma is promising.

In such therapeutic applications of anti-HM1.24 antibody, it is preferred to have a low immunogenicity to humans, and to this end, reshaped human (humanized) antibody of anti-HM1.24 antibody has been developed (WO 98/14580). However, it has been desired to provide a HM1.24 antibody that has a further enhanced ADCC activity and, specifically, a humanized HM1.24 antibody that has an enhanced ADCC activity.

As a method of enhancing the ADCC activity of antibody, alteration of sugar chains of antibody has been known. WO 99/54342, for example, describes modifying the glycosylation of antibody to improve ADCC activity. WO 00/61739 also describes the regulation of ADCC activity by the presence or absence of fucose in antibody sugar chains. WO 02/31140 describes the preparation of an antibody having no α-1,6 core fucose by producing the antibody in the YB2/0 cell. WO 02/79255 describes an antibody having a sugar chain that comprises bisected GlcNAc. However, no HM1.24 antibody has been known in which ADCC activity has been enhanced by the modification of sugar chains.

Patent document 1: WO 98/14580
Patent document 2: WO 99/54342
Patent document 3: WO 00/61739
Patent document 4: WO 02/31140
Patent document 5: WO 02/79255
Non-patent document 1: Ishikawa J. et al., Genomics 26 (1995), 527-534
Non-patent document 2: Goto T. et al., Blood 84 (1994), 1922-1930
Non-patent document 3: Ozaki S. et al., Blood 90 (1997), 3179-3186

DISCLOSURE OF THE INVENTION

Thus, it is an object of the present invention to provide an anti-HM1.24 antibody having an enhanced ADCC activity by the modification of sugar chains, and a method of producing said antibody.

After intensive and extensive study to resolve the above problems, the present inventors have found that an anti-HM1.24 antibody having a sugar chain that contains no α-1,6 core fucose (position 6 of N-acetylglucosamine at reducing end and position 1 of fucose are α-bonded), and an antibody having a sugar chain that has a bisecting N-acetylglucosamine (GlcNAc) structure have a high ADCC activity, and that an anti-HM1.24 antibody having both a sugar chain that contains no α-1,6 core fucose and a sugar chain that has a bisecting N-acetylglucosamine (GlcNAc) structure has a further higher ADCC activity, and therefore have completed the present invention.

Thus, the present invention provides a sugar chain-altered antibody (anti-HM1.24 antibody) against HM1.24 antigen, for example an antibody (anti-HM1.24 antibody) against HM1.24 antigen wherein antibody-dependent cellular cytotoxicity (ADCC) has been enhanced by altering a sugar chain of said antibody. The antibody is typically a monoclonal antibody or an antibody derived therefrom, such as a chimeric antibody, or more preferably a humanized antibody. More specifically, the present invention provides an antibody having a sugar chain that contains no α-1,6 core fucose, an antibody having a sugar chain that has a bisecting N-acetylglucosamine (GlcNAc) structure, and furthermore an antibody having both a sugar chain that contains no α-1,6 core fucose and a sugar chain that has a bisecting N-acetylglucosamine (GlcNAc) structure.

The present invention also provides an antibody composition comprising an anti-HM1.24 antibody having a fucose-free sugar chain and wherein the relative ratio of the fucose-free sugar chain is 30% or more. More preferably, such a relative ratio is 35% or more.

The present invention also provides a method of producing said sugar chain-altered antibody which method comprises culturing YB2/0 cells having introduced therein a nucleic acid encoding an antibody (anti-HM1.24 antibody) against HM1.24 antigen, and harvesting said antibody from said culture; which method comprises culturing a host cell having introduced therein a nucleic acid encoding N-acetylglucosaminyl transferase III (GnTIII), and harvesting said antibody from said culture; and which method comprises culturing YB2/0 cells having introduced therein a nucleic acid encoding N-acetylglucosaminyl transferase III (GnTIII), and harvesting said antibody from said culture.

BRIEF EXPLANATION OF THE DRAWINGS

Fig. 1 shows a SDS-PAGE (12%T) pattern of purified humanized anti-human HM1.24 antibody expressed in YB2/0. The left figure: under a reducing condition, the right figure: under a non-reducing condition. Four μg of purified humanized anti-human HM1.24 antibody was applied.

Fig. 2 is a result of an experiment in which the ADCC activity of human PBMC was determined at each concentration of HM1.24 antibody-DG44 and HM1.24 antibody-YB with four types of HM1.24 antigen-expressing CHO cells (HM26, HM31, HM21, HM36) as the target cell at an E/T ratio=25.

Fig. 3 is a result of an experiment in which the ADCC activity of human PBMC was determined at 1 mg/ml of HM1.24 antibody-DG44 and HM1.24 antibody-YB with HM31 as the target cell at an E/T ratio=1, 5, and 25.

Fig. 4 is a chromatogram of reverse phase HPLC of a pyridylaminated sugar chain prepared from a CHO-derived antibody (a) and a YB2/0-derived antibody (b). It shows that sugar chain patterns are different with different types of producing cells, and specifically in the YB2/0-derived antibody, a group of peaks (A-D) estimated to be fucose-free are increased.

Fig. 5 shows the structures of sugars A-H shown in Fig. 4 and Table 1.

Fig. 6 shows the structures of sugars I-O shown in Fig. 4 and Table 1.

Fig. 7 shows the combination of human GnTIII cDNA with primer sequences used for total synthesis by PCR. PCR fragments with flanking the BamHI sequence that had previously been introduced into primers were ligated at the same sites to obtain the total sequence of human GnTIII cDNA.

Fig. 8 is a comparison of ADCC activity at 100 ng/ml of HM1.24 antibody-DG44 and antibodies derived from clones that produce GnTIII-expressing and producing humanized anti-human HM1.24 antibody. By allowing GnTIII to be expressed, antibody-producing clones having an enhanced ADCC activity were obtained.

Fig. 9 is a result of an experiment in which the ADCC activity of human PBMC was determined at each concentration of HM1.24 antibody-DG44 and humanized anti-human HM1.24 antibody derived from GnTIII-expressing CHO cells with HM36 as the target cell at an E/T ratio=25.

Fig. 10 shows a comparison of a nucleotide sequence (SEQ ID NO: 28) (GnTIII ori.nuc) encoding the native human GnTIII with a nucleotide sequence (SEQ ID NO: 29) (GnTIII mut.nuc) encoding a mutant human GnTIII. In the figure, the asterisk indicates that the corresponding nucleotides in both sequences are the same.

Fig. 11 shows a comparison of a nucleotide sequence (SEQ ID NO: 28) (GnTIII ori.nuc) encoding the native human GnTIII with a nucleotide sequence (SEQ ID NO: 29) (GnTIII mut.nuc) encoding a mutant human GnTIII. In the figure, the asterisk indicates that the corresponding nucleotides in both sequences are the same.

Fig. 12 shows a comparison of a nucleotide sequence (SEQ ID NO: 28) (GnTIII ori.nuc) encoding the native human GnTIII with a nucleotide sequence (SEQ ID NO: 29) (GnTIII mut.nuc) encoding a mutant human GnTIII. In the figure, the asterisk indicates that the corresponding nucleotides in both sequences are the same.

Fig. 13 shows a comparison of a nucleotide sequence (SEQ ID NO: 28) (GnTIII ori.nuc) encoding the native human GnTIII with a nucleotide sequence (SEQ ID NO: 29) (GnTIII mut.nuc) encoding a mutant human GnTIII. In the figure, the asterisk indicates that the corresponding nucleotides in both sequences are the same.

Fig. 14 shows a comparison of a nucleotide sequence (SEQ ID NO: 28) (GnTIII ori.nuc) encoding the native human GnTIII with a nucleotide sequence (SEQ ID NO: 29) (GnTIII mut.nuc) encoding a mutant human GnTIII. In the figure, the asterisk indicates that the corresponding nucleotides in both sequences are the same.

Fig. 15 shows a comparison of a nucleotide sequence (SEQ ID NO: 28) (GnTIII ori.nuc) encoding the native human GnTIII with a nucleotide sequence (SEQ ID NO: 29) (GnTIII mut.nuc) encoding a mutant human GnTIII. In the figure, the asterisk indicates that the corresponding nucleotides in both sequences are the same.

BEST MODE FOR CARRYING OUT THE INVENTION

In accordance with the present invention, antibody (sugar chain-altered antibody) of which sugar chain has been altered means an antibody that, when compared with sugar chain of the antibody (preferably the antibody that is produced in the highest proportion among the antibodies produced by such a reference host cell) produced by the reference host cell, has a different sugar chain structure, and comprises antibodies having sugar chain structures that are not normally (or predominantly) produced by the reference host cell. Also, when the sugar chain-altered antibodies of the present invention are considered an assembly (also referred to herein as the antibody composition) of antibody molecules having various sugar chains that not always uniform, it means an antibody composition having different proportions of sugar chain-altered antibodies as compared to the antibody composition produced by the reference host cell, and such antibody composition of the present invention also includes antibody compositions that contain antibodies having sugar chain structures that are not normally (or predominantly) produced by the reference host cell.

The reference host cell includes, but is not limited to, CHO dhfr- cells (ATCC CRL-9096), CHO K1 (ATCC CCL-61), CHO DG44 and the like, and preferably the CHO DG44 cell is used as the reference host cell.

As examples of sugar chain-altered antibodies, there can be mentioned fucose-deficient antibodies having sugar chains in which fucose (preferably α-1,6 core fucose) is deficient, antibodies in which bisecting N-acetylglucosamine (GlcNAc) has been added to the sugar chain thereof, and the like.

When the sugar chain-altered antibody of the present invention is considered as an antibody composition, it does not need to be a composition that contains homogeneous antibodies having sugar chains of the identical structure, and thus, in accordance with the present invention, the proportion of the sugar chain-altered antibodies contained in the antibody composition only needs to be different from that of the sugar chain-altered antibodies contained in the antibody composition produced by the above reference host cell. The present invention includes compositions of such sugar chain-altered antibodies. The identity of the composition of sugar chain-altered antibodies of the present invention can be confirmed based on whether the proportion is different from that of the sugar chain-altered antibodies contained in the reference antibody composition described above. Such a proportion can be compared by the relative proportion of each sugar chain recognized by the analytical method disclosed in, for example, Working Example 8 described below.

In order to obtain anti-HM1.24 antibody of the present invention in which the ADCC activity has been enhanced by the modification of sugar chains, it is necessary to express anti-HM1.24 antibody in a host cell having no or low ability of adding α-1,6 core fucose or to express anti-HM1.24 antibody in a host cell having an ability of forming a bisecting N-acetylglucosamine (GlcNAc) structure on a sugar chain. To that end, a gene encoding the desired anti-HM1.24 antibody must be cloned. Anti-HM1.24 antibodies encoded by the cloned gene include, for example, monoclonal antibodies, chimeric antibodies in which the variable region is derived from an animal other than the human and the constant region is derived from a human antibody, humanized antibodies in which the complementarity determining region alone of the variable region is derived from an antibody of an animal other than the human and the other regions of the antibody are derived from a human antibody, and the like.

As can be seen from the description in WO 98/14580, the hybridoma that produces the monoclonal anti-HM1.24 antibody has already been established and has been deposited on April 27, 1995 with the Patent Microorganism Depository, the National Institute of Bioscience and Human Technology (Chuo Dai 6, 1-1, Higashi 1-chome, Tsukuba city, Ibaraki Pref., Japan) as FERM BP-5233. From this hybridoma, DNA encoding a light chain variable region (L chain V region) and DNA encoding a heavy chain variable region (H chain V region) have been cloned, and E. coli having the plasmids containing these DNA's have been deposited on August 29, 1996 with the Patent Microorganism Depository, the National Institute of Bioscience and Human Technology (Chuo Dai 6, 1-1, Higashi 1-chome, Tsukuba city, Ibaraki Pref., Japan) as Escherichia coli DH5α (pUC19-1.24L-gκ) (FERM BP-5646) and Escherichia coli DH5α (pUC19-1.24H-gγ1) (FERM BP-5644), respectively. Furthermore, from the above cloned DNA encoding the L chain V region and DNA encoding the H chain V region, chimeric anti-HM1.24 antibody and humanized anti-HM1.24 antibody were created. For the humanized antibody, as shown in Table 1 to Table 4 on pages 37-40 in WO 98/14580, versions a and b were created for the L chain of the humanized antibody and versions a to s were created for the H chain, and from the measurement of antigen-binding activity of humanized antibodies created by combining them, it was confirmed, humanized antibodies comprising the combination of the L chain version a and the H chain version r or s produce a potent antigen-binding activity.

Thus, in accordance with the present invention, various monoclonal antibodies, chimeric antibodies, humanized antibodies etc. described in WO 98/14580 cited above can be used. In addition to the above antibodies, however, there can be used chimeric antibodies, humanized antibodies etc. derived from other monoclonal antibodies against HM1.24. As methods of preparation in such cases, there can be used the one described in, for example, WO 98/14580.

Sugar chains that bind to anti-HM1.24 antibody include N-glycoside-linked sugar chains that bind to the side chain N atom of asparagine of the antibody molecule, and O-glycosyl-linked sugar chains that bind to the side chain hydroxyl group of serine or threonine of the antibody molecule, and the side chains of which presence or absence concerns in the present invention are the N-glycoside-linked sugar chains. The N-glycosyl-linked sugar chains, as shown in Fig. 5 and 6, have a basic structure (core) [-Manβ1-4GlcNAcβ1-4GlcNc-] in which one mannose (Man) and two N-acetylglucosamines (GlcNAc) are bound with the β1,4-linkage, and GlNAc on the right of the structure is referred to as the reducing terminal and Man on the left is referred to as the non-reducing terminal. When fucose (Fuc) is bound, N-acetylglucosamine on the position 6 of the reducing terminal and the position 1 of fucose are mainly α-linked.

According to one aspect of the present invention, anti-HM1.24 antibody has a sugar chain that does not contain the above fucose. When an antibody molecule has a plurality of N-glycosyl sugar chains, at least one sugar chain does not have the above fucose. Such an antibody having a fucose-free sugar chain may be produced by expressing said antibody in a cell deficient of an ability of adding fucose to the sugar chain or a host that has no or low fucose-transferring ability.

In accordance with the present invention, any cells that have no or low fucose-transferring ability can be used and, as a specific example, there can be mentioned the rat myeloma YB2/3HL.P2.G11.16Ag.20 cells (abbreviated as the YB2/0 cell) (stored as ATCC CRL 1662). Other cells that can be used in this invention include, for example, the FTVIII knock-out CHO cell (WO 02/31140), and the Lecl3 cell (WO 03/035835), the fucose transporter-deficient cell (Patent application No. 2003-174006, Patent application No. 2003-282081, Patent application No. 2003-174010, Patent application No. 2003-282102).

According to another aspect of the present invention, the anti-HM1.24 antibody of the present invention has a sugar chain that has bisecting N-acetylglucosamine. N-glycosyl-linked sugar chains have the basic structure (core) as described above and, on the non-reducing terminal thereof, as shown in Fig. 5, two chains containing mannose are bound with a $\alpha$1,6-linkage and a $\alpha$1,3-linkage. On the other hand, in the sugar chain shown in Fig. 6, one N-acetylglucosamine (GlcNAc) is bound with a $\beta$1,4-linkage in addition to the above two sugar chains on the non-reducing terminal of the basic structure (core). This N-acetylglucosamine (GlcNAc) is the "bisecting N-acetylglucosamine."

Sugar chains having bisecting N-acetylglucosamine are O-glycosyl-linked sugar chains or N-glycosyl-linked sugar chains, and are formed by transferring N-acetylglucosamine to sugar chains with N-acetylglucosaminyl transferase III (GnTIII). A gene encoding this enzyme has already been cloned, and the amino acid sequence and the nucleotide sequence of DNA encoding it have been described (NCBI database (ACCESSION D13789)). This DNA can also be cloned according to a standard method such as the PCR method based on the above sequence information.

In order to form sugar chains that have bisecting N-acetylglucosamine using DNA encoding GnTIII, a host cell that produces anti-HM1.24 antibody may be transformed with an expression vector comprising this DNA. Thus, an expression vector comprising DNA encoding GnTIII and an expression vector comprising DNA encoding anti-HM1.24 antibody are used to transform a host cell, which is then cultured.

According to the third aspect of the present invention, the anti-HM1.24 antibody of the present invention has both a sugar chain that does not have $\alpha$-1,6 core fucose and a sugar chain that has bisecting N-acetylglucosamine. In order to produce this type of antibody, an expression vector comprising DNA encoding GnTIII and an expression vector comprising DNA encoding anti-HM1.24 antibody are used to transform a host cell, such as the YB2/0 cell, that has no or weak activity of forming a sugar chain having $\alpha$-1,6 core fucose, and then the cell is cultured.

Transformation of host cells, culturing, and isolation and purification of antibody from the culture may be carried out according to standard methods.

Preferably, the sugar chain-altered antibody of the present invention has an enhanced ADCC activity. With regard to whether ADCC activity has been enhanced or not, in accordance with the present invention, ADCC activity is judged to be enhanced when it is higher than that of an antibody in which sugar chains have not been altered or an antibody composition in which sugar chains have not been altered.

ADCC activity may be determined by a method known to a person skilled in the art. In particular, it can be determined by mixing, for example, an effector cell, a target cell and anti-HM1.24 antibody, and then determining the degree of ADCC thereof. More specifically, for example, mouse splenocytes or mononuclear cells derived from human peripheral blood or bone marrow may be used as the effector cell, and CHO cells that express HM1.24 antigen may be used as the target cell. To the target cells that had previously been labelled with $^{51}$Cr, effector cells are added at an appropriate ratio to the target cells, which are then incubated. After incubation, the supernatant is harvested, and radioactivity in the supernatant may be counted to determine ADCC activity.

## EXAMPLES

The present invention will now be explained more specifically with reference to the following examples.

Example 1. Expression of humanized anti-human HM1.24 antibody in rat myeloma YB2/0

Ten $\mu$g of a vector expressing humanized anti-human HM1.24 antibody (AHi/N5KG1V-lark, Barnett, R.S. et al., Antibody Production in Chinese Hamster Ovary Cells Using Impaired Selectable Marker. In: Wang, H.Y. & Imanaka, T. (eds) ACS Symposium Series Vol 604: Antibody Expression and Engineering, 27, 1995, WO 98/4580) was introduced into 2x$10^6$/0.6 ml PBS(-) of YB2/0 (ATCC CRT-1662) by electroporation at a condition of 1.5 kV and 25$\mu$F. Culturing was carried out at 37°C in a 5% CO2 incubator.

After 400 $\mu$g/ml of Geneticin was added to the RPMI 1640 medium (Gibco) containing 10% FCS for selection, a gene was amplified at a sequentially increasing concentration of 50 nM MTX, 100 nM MTX, and 200 nM MTX. Also 0.5 cells/100 μL/well was plated into a 10% FCS/RPMI 1640 containing 200 nM MTX and 400 μg/ml of Geneticin in a 96-well plate (Falcon) in order to clone cells by the limiting dilution method.

The culture supernatant of the YB2/0 cells into which the.gene of humanized anti-human HM1.24 antibody had been introduced was determined by ELISA shown in Example 2.

Example 2. Determination of humanized anti-HM1.24 antibody (ELISA method)

To a 96-well ELISA plate (manufactured by Nunc), soluble HM1.24 antigen diluted to about 100 ng/ml with a coat buffer (100 mmol/L sodium hydrogen carbonate, pH 9.6) was added in portions of 100 μL, and then incubated, at 4°C, overnight or longer. After incubation, 1% BSA-PBS was added at 200 μL/well and then allowed to stand at room temperature for about two hours, and the prepared plate was stored at 4°C. After 1% BSA-PBS was decanted off, each well was washed with Tween-PBS.

Appropriately diluted humanized anti-HM1.24 antibody standard solutions or sample solutions and biotin-labelled humanized anti-HM1.24 antibody diluted to 100 ng/ml were mixed at 1:1, and then aliquoted at 100 μL/well. After incubating for 1 hour at room temperature, each well was washed with Tween-PBS. Avidin-labelled HRP was added to each well, incubated at room temperature for 15 minutes or longer, TMB liquid (manufactured by Sigma) was added thereto at 100 μL/well, and 50 μL/well of 2 mol/L sulfuric acid was added to stop the reaction, and then the absorbance at 450 nm was measured. From the calibration curve of concentration-absorbance for humanized anti-HM1.24 antibody standard solutions, the humanized HM1.24 antibody concentration of sample solutions was calculated.

Example 3. Purification of humanized anti-human HM1.24 antibody expressed in YB2/0

Cells for which the expression of humanized anti-human HM1.24 antibody was confirmed were subjected to an expansion culture with 1700 $cm^2$ of a roller bottle (CORNING). Thus, $1\times10^9$ humanized anti-human HM1.24 antibody-expressing YB2/0 cells were cultured to confluence in a 400 ml of 10% FCS/RPMI 1640 medium containing 200 nM MTX and 400 μg/ml of gentamycin (2.5 rpm). Then, FCS was passed through rProtein A FF (Amersham Pharmacia) equalized with PBS(-) for collection of the culture supernatant to remove bovine-derived IgG (FCS(-)), and this FCS(-) was cultured in a 10% FCS(-)/RPMI 1640 medium containing 200 nM MTX and 400 μg/ml of geneticin for 3-4 days.

After the culture supernatant was treated with a 0.22 μm filter, it was purified with rProtein A FF (PBS/PBS-citric acid: linear gradient elution) and Source 15S (20 mM acetic acid, 0-0.5 mM NaCl: linear gradient elution). The purified humanized anti-human HM1.24 antibody was termed as HM1.24 antibody-YB (Fig. 1).

Example 4. Preparation of CHO cells expressing HM1.24 antigen (BST-2)

CHO cells expressing the HM1.24 antigen protein were prepared as follows (Ohtomo T. et al., Biochemical and Biophysical Research Communications 258 (1999), 583-591). Thus, an expression vector p3.19 (see supra) encoding HM1.24 antigen was introduced to a DHFR-deficient CHO cell line, 500 μg/ml of G418 was used for selection, and the limiting dilution was performed to obtain four cell lines HM26, HM31, HM21 and HM36. The number of the HM1.24 antigen expressed on the cell surface, as determined by flow cytometry in a method described in Patent application No. 2001-115889, was $3.8x10^3$, $2.2x10^4$, $2.2x10^4$, and $1.8x10^5$, respectively.

Example 5. Determination of ADCC activity usin human peripheral blood-derived PBMC

(1) Preparation of human PBMC solution

Peripheral blood drawn with heparin from a normal healthy subject was diluted by two-fold with PBS(-), and layered on Ficoll-Paque™ PLUS (Amersham Pharmacia Biotech AB). After centrifuging this (500xg, 30 minutes, 20°C), the interlayer, a mononuclear cell fraction, was collected. After washing three times, it was suspended into 10% PBS/RPMI to prepare the human PBMC solution.

(2) Preparation of the target cell solution

CHO cells that express HM1.24 antigen (BST-2) described in Example 4 were peeled from the dish using the cell detachment buffer (Invitrogen Corp), and were suspended in 200 μl of 10% FBS/RPMI, to which 5.55 MBq of chromium-51 was added, and incubated at 37°C for one hour in a 5% carbon dioxide incubator. After washing the cells three times, they were prepared into an individual cell concentration in 10% FBS-RPMI 1640 medium to prepare the target cell solutions.

(3) Chromium release test (ADCC activity)

After the target cell solutions were aliquoted in 50 μl portions into a 96-well U-bottomed plate, 50 μl each of antibody solutions prepared at each concentration was added thereto, and incubated on ice for one hour, then 100 μl of the human PBMC solution was added, incubated at 37°C for four hours in a 5% carbon dioxide incubator, and then the radioactivity of 100 μl of the culture supernatant after incubation was determined by a gamma counter. The specific chromium release rate was determined based on the following formula:

$$\text{Specific chromium release rate (\%)} = (A-C)\times 100/(B-C)$$

A represents a mean of radioactivity (cpm) for each well, B represents a mean of radioactivity (cpm) of a well in which 50 μl of a target cell suspension, 20 μl of a 10% NP-40 aqueous solution (Nonidet™ P-40, manufactured by Nacalai Tesque Inc.) and 130 μl of a 10% FBS/RPMI medium were added, and C represents a mean of radioactivity (cpm) of a well in which 50 μl of a target cell suspension and 150 μl of a 10% FBS/RPMI medium were added.

Example 6. Method of determining ADCC activity using a CHO cell line that stably expresses β-galactosidase

As the effector cell, mononuclear cells isolated from the peripheral blood of a normal healthy subject by the density centrifugation method was used. Thus, an equal amount of PBS was added to the peripheral blood of the normal healthy subject, layered on Ficoll-Paque PLUS (Pharmacia), and then centrifuged at 500g for 30 minutes. The mononuclear cell phase was collected, washed three times with RPMI 1640 containing 10% FCS, and then prepared to a cell count of $5\times10^6$/ml with α-MEM containing 10% FCS.

After detaching with trypsin-EDTA, 50 μl of the CHO#30 cell line that stably expresses β-galactosidase suspended at $2\text{x}10^5$ cells/ml in α-MEM containing 10% FCS and 50 μl of various concentrations of anti-HM1.24 antibody were added into a 96-well U-bottomed plate, which were incubated at 4°C for 15 minutes. Then 100 μl of the effector cells was added and incubated at 37°C for four hours. After incubation, 20 μl of the culture supernatant was collected, and the β-galactosidase activity thereof was determined. The maximum amount of the released enzyme was set as the amount of enzyme released with a cell lysis buffer of the Galactone-star assay kit.

Cytotoxic activity was calculated as:

$$\text{Cytotoxic activity (\%)} = (A-C)\times 100/(B-C)$$

wherein, A represents the activity (RLU/sec) of P-galactosidase released in the presence of the antibody, B represents the activity (RLU/sec) of β-galactosidase released with the cell lysis buffer, and C represents the activity (RLU/sec) of β-galactosidase released with the culture liquid alone without antibody.

Example 7. Determination of ADCC activity of YB2/0-derived humanized anti-human HM1.24 antibody

The ADCC activity of HM1.24 antibody (HM1.24 antibody-YB) expressed in YB2/0 determined by the method described in Example 5 is shown in Fig. 2 to Fig. 3. For any target cells, as shown in Fig. 2, HM1.24 antibody-YB exhibited a higher ADCC activity than the HM1.24 antibody (HM1.24 antibody-DG44) produced in DG44 (DHFR-deficient CHO cells: Urlaub, G. et al. (1986) Effect of Gamma Rays at the Dihydrofolate Reductase Locus: Deletions) and Inversions. Somatic Cell and Molecular Genetics, 12: 555, 1986).

Specifically, the induction of ADCC activity was noted at lower concentrations, and the maximal ADCC activity also increased. In particular, when target cells HM26 and HM31 that express small numbers of HM1.24 antigen were used, HM1.24 antibody-DG44 exhibited very low ADCC activity whereas HM1.24 antibody-YB exhibited high ADCC activity. Also, as shown in Fig. 3, not only when the ratio (E/T ratio) of the PBMC count relative to that of the target cells was 25 but when the E/T ratio was 5, HM1.24 antibody-YB exhibited a higher ADCC activity than HM1.24 antibody-DG44.

Example 8. Analysis of sugar chains

1. Preparation of 2-aminopyridine-labelled sugar chains (pyridylaminated sugar chains)

N-Glycosidase (Roche) reacted to the YB2/0-derived antibody of the present invention and the CHO-derived antibody as the control sample to release sugar chains from the protein (Weitzhandler M. et al., Journal of Pharmaceutical Sciences 83:12 (1994), 1670-1675). After the sugar chains were desalted by solid-phase extraction using a cellulose cartridge (manufactured by TAKARA) (Shimizu Y. et al., Carbohydrate Research 332 (2001), 381-388), they were concentrated to dryness, and then fluorescently labelled with 2-aminopyridine (Kondo A. et al., Agricultural and Biological Chemistry 54: 8 (1990), 2169-2170). After the pyridylaminated sugar chains obtained were desalted by solid-phase extraction using a cellulose cartridge, they were concentrated by centrifugation to prepare purified pyridylaminated sugar chains.

2. Analysis by reverse phase HPLC of purified pyridylaminated sugar chains

After pyridylaminated sugar chains were prepared for the YB2/0-derived antibody of the present invention and the CHO-derived antibody as the control sample in the method of the above Working Example 8-1, reverse phase HPLC analysis was performed with an ODS column (Palpak Type R manufactured by TAKARA) and the chromatograms were compared. As compared to the sugar chains of the CHO-derived antibody, it was confirmed, the sugar chains of the YB2/0-derived antibody exhibited increases in peaks of sugar chains (A-D), possibly fucose-free, that eluate at 20-35 minutes (Fig. 4).

3. Analysis by two dimensional mapping of purified pyridylaminated sugar chains

After pyridylaminated sugar chains were prepared for the YB2/0-derived antibody of the present invention in the method of the above Example 8-1, the two dimensional mapping that combined reverse phase HPLC with an ODS column and a normal phase HPLC with an amine column (Palpak Type N manufactured by TAKARA) was performed. Specifically, the normal phase HPLC with an amine column is used to roughly fractionate the purified pyridylaminated sugar chains, and then each fraction is analyzed with the reverse phase HPLC.

Each sugar chain was identified by comparing its elution position in HPLC with those of the pyridylaminated sugar chain standards (manufactured by TAKARA, manufactured by Hohnen, manufactured by Seikagaku Kogyo; excluding K, O, and P in Fig. 5) and confirming molecular weight by TOF-MS. The relative ratio of each sugar chain identified is shown in Table 1 (separation of J from K and separation of N from O have not been performed in this Example). The structures of sugar chains are shown in Fig. 5 and Fig. 6. The result confirmed that in the YB2/0-derived antibody of the present invention, there are 30% or more of fucose-free sugar chains and there are sugar chains that have bisecting GlcNAc.

Table 1

| Sugar chain | Group | Relative ratio of each sugar chain | Relative ratio of each group |
|---|---|---|---|
| A | -Fuc,-Bisecting GlcNAc | 17.7% | 33.5% |
| B | | 9.9% | |
| C | | 3.9% | |
| D | | 1.9% | |
| E | +Fuc,-Bisecting GlcNAc | 22.9% | 55.2% |
| F | | 21.4% | |
| G | | 5.5% | |
| H | | 5.4% | |
| I | -Fuc,+Bisecting GlcNAc | 2.0% | 3.3% |
| J(K) | | 1.3% | |
| M | +Fuc,+Bisecting GlcNAc | 3.7% | 8.0% |
| N(O) | | 4.2% | |

Example 9. Creation of human GnTIII-expressing expression vector

The sequence of the human GnTIII gene was obtained from the NCBI database (ACCESSION D13789). The sequence was analyzed with GENETYX-SV/RC and it was found to have many repeat sequences. In order to facilitate amplification by PCR, primers that had silent mutations in several sites were designed and were obtained by synthesis using PCR. PCR used KOD polymerase (TOYOBO), double strands from the nucleotide numbers 801 to 870 as the initial templates, and the following primers sequentially to perform PCR. In the primer sequences below, bold letters indicate nucleotides in which silent mutations have been introduced. Also, numbers indicate positions from the translation initiation site. Fig. 7 shows the position of each primer relative to the GnTIII gene.

Forward primer

Initial (BamHI) :TTTCTCGAGatgagacgctacaagctctttctcatgttc

1-97: atgagacgctacaagctctttctcatgttctgtatggccggcctgtgcctcatctccttcctgcacttcttcaagaccctgtcctatgtcaccttcc

78-177:cctgtcctatgtcaccttcccAcgagaactggcctccctcagccctaacctggtgtccagctttttctggaacaatgccccggtcacgccccaggccagc

158-259:cggtcacgccccaggccagcccTgagccaggaggccctgacctgctgcgtaccccactctactcccactcgcccctgctgcagccgctgccgcccagcaagg

239-331: agccgctgccgcccagcaaggcggccgaggagctccaccgggtggacttggtgctgcccgaggacaccaccgagtatttcgtgcgcaccaagg

312-409: gtatttcgtgcgcaccaaggcTggAggcgtctgcttcaaacccggcaccaagatgctggagagAccgccTccgggacgAccggaggagaagcctgagg

390-472: AccggaggagaagcctgaggggccaacggAtcctcggcCcggcgAccaccccggtacctcctgagcgcccgggagcgcacgg

453-556: gagcgcccgggagcgcacggggggccgaggTgcAcgAcgcaagtgggtggagtgcgtgtgTctgcccggAtggcacggacccagctgcggcgtgcccactgtgg

535-618: agctgcggcgtgcccactgtggtgcagtaTtccaacctgccTaccaaggagcggctggtgcccagggaggtgccgcgccgcgtc

598-696: agggaggtgccgcgccgcgtcatTaaTgcTatcaacgtcaaccacgagttcgacctgctggacgtgcgcttccacgagctgggcgacgtggtggacgcc

677-777: tgggcgacgtggtggacgcctttgtggtgtgcgagtccaacttcacggcttatggggagccgcggccgctcaagttccgggagatgctgaccaatggcacc

758-820: agatgctgaccaatggcaccttcgagtacatccgccacaaggtgctctatgtcttcctggacc

801-870: gctctatgtcttcctggaccactttTccTccTggAggAcgAcaAgaTggAtggatcgccgacgactacctg

Reverse primer

End(HindIII) :TTTAAGCTTActagacttccgcctcgtccagtttTcc

1596-1488: ctagacttccgcctcgtccagtttTccccgAgcAggcggTcttccTtcAggacccctgtggcgccaTccTcccgcAgccgtgctcctgggctcctggtaggggttgtcc

1508-1407:ggctcctggtaggggttgtccagAaggtagtggaaccggtcgtagttcttcagcaggtacttgggcgcatacatgtgctcgctggggtctgcaggcgggtac

1427-1324: ctggggtctgcaggcgggtactcTtgctgcgtgccgtcgaaccagcccccggtgcggatcaggccgcggatgtagttcaggtcccgcttgtcctcgtagtcacc

1344-1244: ccgcttgtcctcgtagtcaccccagcgtgggaagtcgccattctgggcggacacgagcttgaagtagatgccctcgggcgtgaagcaccaggagcagtgcc

1264-1162: tgaagcaccaggagcagtgccagccggcgaagtgAagggggctgcccagcgaccactgcaccaggatgtgTccggtgcggttctcatactgtctgaagttgg

1182-1084: ctcatactgtctgaagttgggcatggtgtagtaTtggcggcggcgcaggcggatgccgtccagcccatacactgcctgcagcatgtccaccgtgcagcc

1103-1004: agcatgtccaccgtgcagcctgacaccacctccagggtgcccggTtgcttccaAaagaaTccgtagagcgacgtgcgcatgtggaaggcgaagggctcgg

1023-922: gtggaaggcgaagggctcggtccagccatcgtagagcttgaggaaCaggacgccgtcacgggccgggatctcgtccgcatcgtcaatgatgaagacgtcgtc

941-851: tcaatgatgaagacgtcgtcgggccgcaggttgcgcagccgcgagacgccgtcctgggtgaggaaggtgcgcaggtagtcgtcggcgatcc

870-801: caggtagtcgtcggcgatccaTccAtcTtgTcgTccTccAggAggAaagtggtccaggaagacatagagc

380-300 ggcggTctctccagcatcttggtgccgggtttgaagcagacgccTccAgccttggtgcgcacgaaatactcggtggtgtcc

320-241 acgaaatactcggtggtgtcctcgggcagcaccaagtccacccggtggagctcctcggccgccttgctgggcggcagcgg

Bam-359 TTTggaTccgttggccccctcaggcttctcctccggTcgtcccggAggcggTctctccagcatcttgg

As needed, amplified fragments were subjected to agarose gel electrophoresis, and fragments of interest were excised from the gel and purified, and used as the template for the subsequent PCR. Since it was finally impossible to amplify the full-length by PCR alone, BamHI sites that had previously been introduced into the primer as silent mutation were used, and fragments flanking the sites were ligated after amplification to obtain the full-length human GnTIII gene. Fig. 11 to Fig. 15 show a comparison of a nucleotide sequence (SEQ ID NO: 28) (GnTIII ori.nuc) encoding the native human GnTIII with a nucleotide sequence (SEQ ID NO: 29) (GnTIII mut.nuc) encoding a mutant human GnTIII. In the figure, the asterisk indicates that the corresponding nucleotides in both sequences are the same.

Human GnTIII was integrated into the XhoI/HindIII site of pcDNA3.1(Hygro-) (Invitrogen) and the sequence was confirmed.

Example 10. Expression of GnTIII in CHO cells that express HM1.24 antibody-DG44

Ten μg of GnTIII/pcDNA3.1(Hygro-) obtained in the above Example 9 was introduced into the HM1.24 antibody-DG44-expressing CHO line by electroporation under a condition of 1.5 kV and 25μF. Culturing was carried out in a 5% CO2 incubator at 37°C. Using the IMDM medium (Gibco) containing 10% FCS, 10 cells/100 μL/well were plated in a 96-well plate (Falcon), and cultured for two days. The medium was replaced with a 10% FCS/IMDM medium containing 400 μg/ml hygromycin and cells were selected for 1-2 weeks. The culture supernatant of the cells for which hygromycin-resistant colonies developed and growth was noted was collected, and the amount of humanized anti-human HM1.24 antibody was determined by the ELISA method described in Example 2.

Example 11. Screening of humanized anti-human HM1.24 antibody-producing CHO cells that express GnTIII

The cultured medium of humanized anti-human HM1.24 antibody derived from humanized anti-human HM1.24 antibody-producing cells (clones No. 1-31) in which GnTIII was forcefully expressed and HM1.24 antibody-DG44 were diluted with the medium to an antibody concentration of 400 ng/ml, and ADCC activity was determined using the method described in Example 6 and compared (Fig. 8).

Finally, screening was carried out taking into account ADCC activity and the amount of humanized anti-human HM1.24 antibody expressed and the growth rate, and the clone No. 6 (57B2) was obtained.

Example 12. Determination of ADCC activity of humanized human HM1.24 antibody derived from GnTIII-expressing CHO cells

The ADCC activity of humanized anti-human HM1.24 antibody, derived from humanized anti-human HM1.24 antibody-producing cells in which GnTIII was forcefully expressed, was determined in the method described in Example (B) and the result is shown in Fig. 9. Clone No.3 and No. 6 (57B2) were compared to HM1.24 antibody-DG44 with a result that any of the clones exhibited higher ADCC activity than HM1.24 antibody-DG44. Industrial Applicability

In accordance with the present invention, anti-HM1.24 antibody having a high ADCC activity can be produced.

## Claims

1. A sugar chain-altered antibody (anti-HM1.24 antibody) against HM1.24 antigen.

2. The antibody (anti-HM1.24 antibody) against HM1.24 antigen according to claim 1 in which the alteration of sugar chains resulted in enhanced antibody-dependent cellular cytotoxicity (ADCC).

3. The antibody according to claim 1 or 2 in which said antibody is a monoclonal antibody.

4. The antibody according to claim 1 or 2 in which said antibody is a chimeric antibody.

5. The antibody according to claim 1 or 2 in which said antibody is a humanized antibody.

6. The antibody according to any one of claims 1-5 having a sugar chain that does not contain $\alpha$-1,6 core fucose.

7. The antibody according to any one of claims 1-5 having a sugar chain that contains a bisecting N-acetylglucosamine (GlcNAc) structure.

8. The antibody according to any one of claims 1-7 having a sugar chain that does not contain $\alpha$-1,6 core fucose and a sugar chain that contains a bisecting N-acetylglucosamine (GlcNAc) structure.

9. An antibody composition comprising anti-HM1.24 antibody having a fucose-free sugar chain, wherein the relative ratio of the fucose-free sugar chain is 30% or more.

10. A method of producing said antibody according to claim 6 which method comprises culturing cells deficient in fucose-adding ability having introduced therein a nucleic acid encoding an antibody (anti-HM1.24 antibody) against HM1.24 antigen, and harvesting said antibody from said culture.

11. A method of producing said antibody according to claim 7 which method comprises culturing a host cell having introduced therein a nucleic acid encoding N-acetylglucosaminyl transferase III (GnTIII), and harvesting said antibody from said culture.

12. A method of producing said antibody according to claim 8 which method comprises culturing cells deficient in fucose-adding ability having introduced therein a nucleic acid encoding N-acetylglucosaminyl transferase III (GnTIII), and harvesting said antibody from said culture.

Fig.1

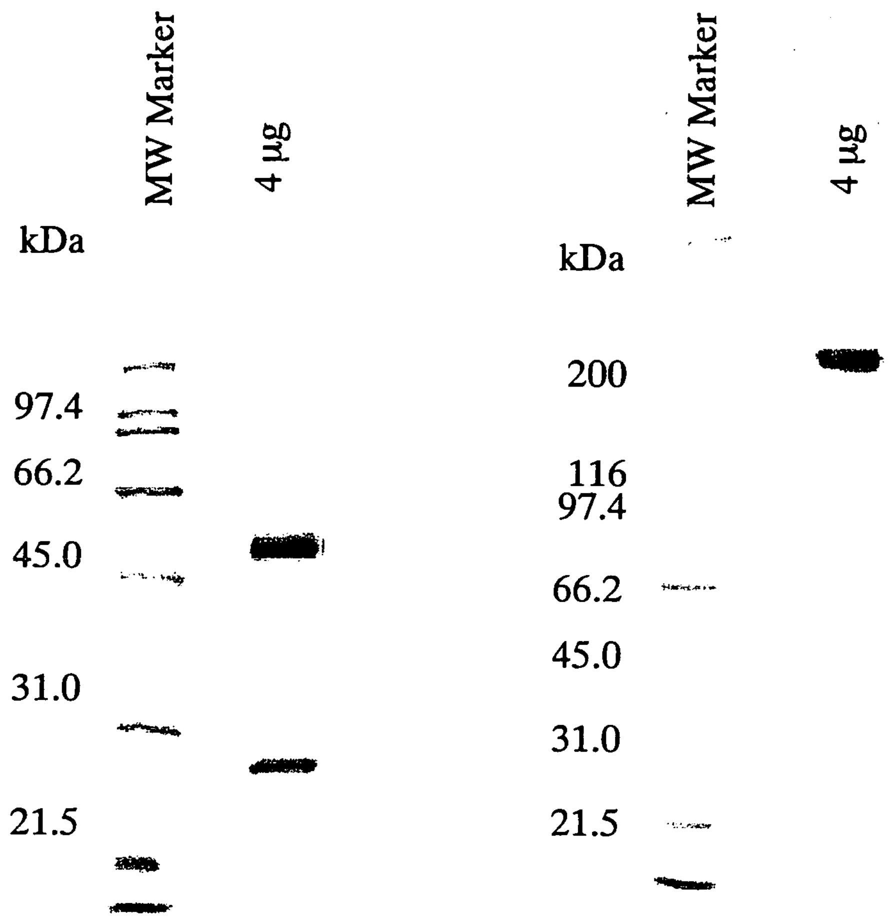
MW Marker
4 μg
kDa
97.4
66.2
45.0
31.0
21.5
MW Marker
4 μg
kDa
200
116
97.4
66.2
45.0
31.0
21.5

Fig.2

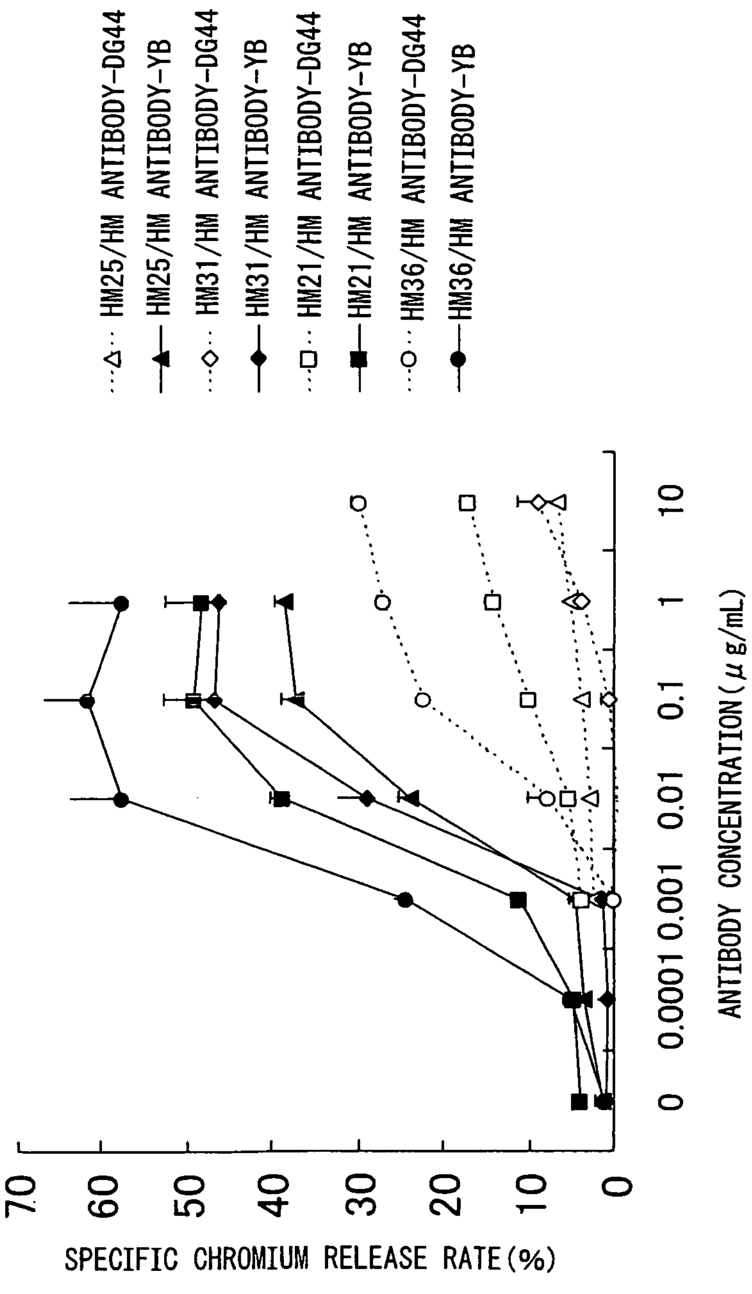
HM25/HM ANTIBODY-DG44
HM25/HM ANTIBODY-YB
HM31/HM ANTIBODY-DG44
HM31/HM ANTIBODY-YB
HM21/HM ANTIBODY-DG44
HM21/HM ANTIBODY-YB
HM36/HM ANTIBODY-DG44
HM36/HM ANTIBODY-YB
SPECIFIC CHROMIUM RELEASE RATE(%)
70
60
50
40
30
20
10
0
0
0.0001
0.001
0.01
0.1
1
10
ANTIBODY CONCENTRATION (μg/mL)

Fig.3

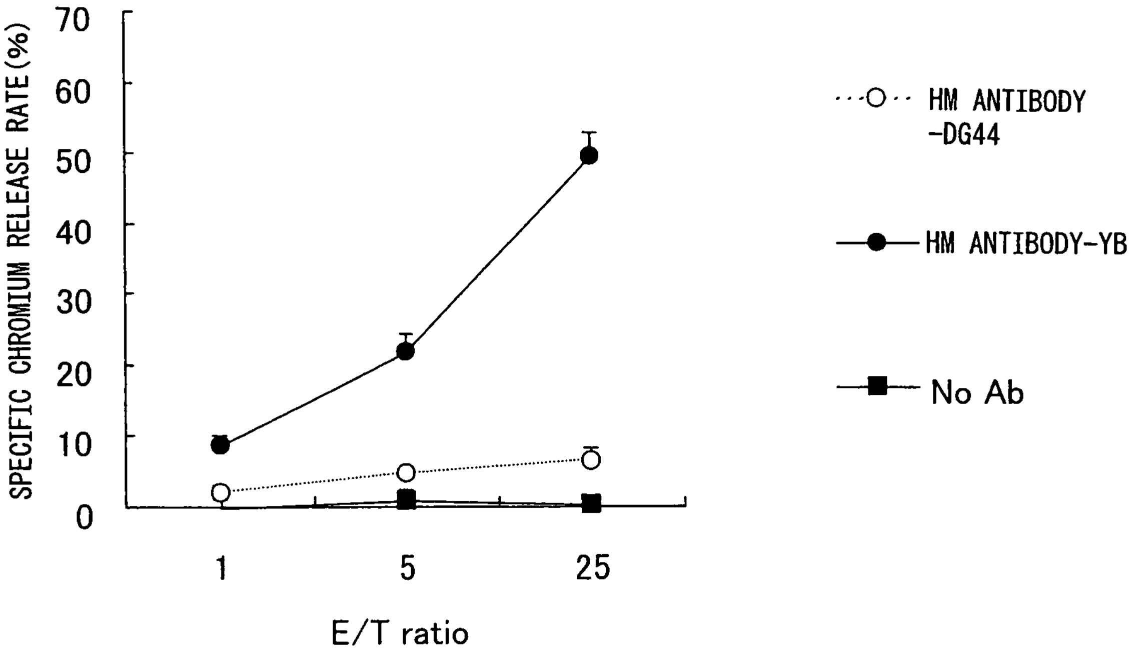
SPECIFIC CHROMIUM RELEASE RATE (%)
70
60
50
40
30
20
10
0
1
5
25
E/T ratio
HM ANTIBODY -DG44
HM ANTIBODY-YB
No Ab

Fig.4

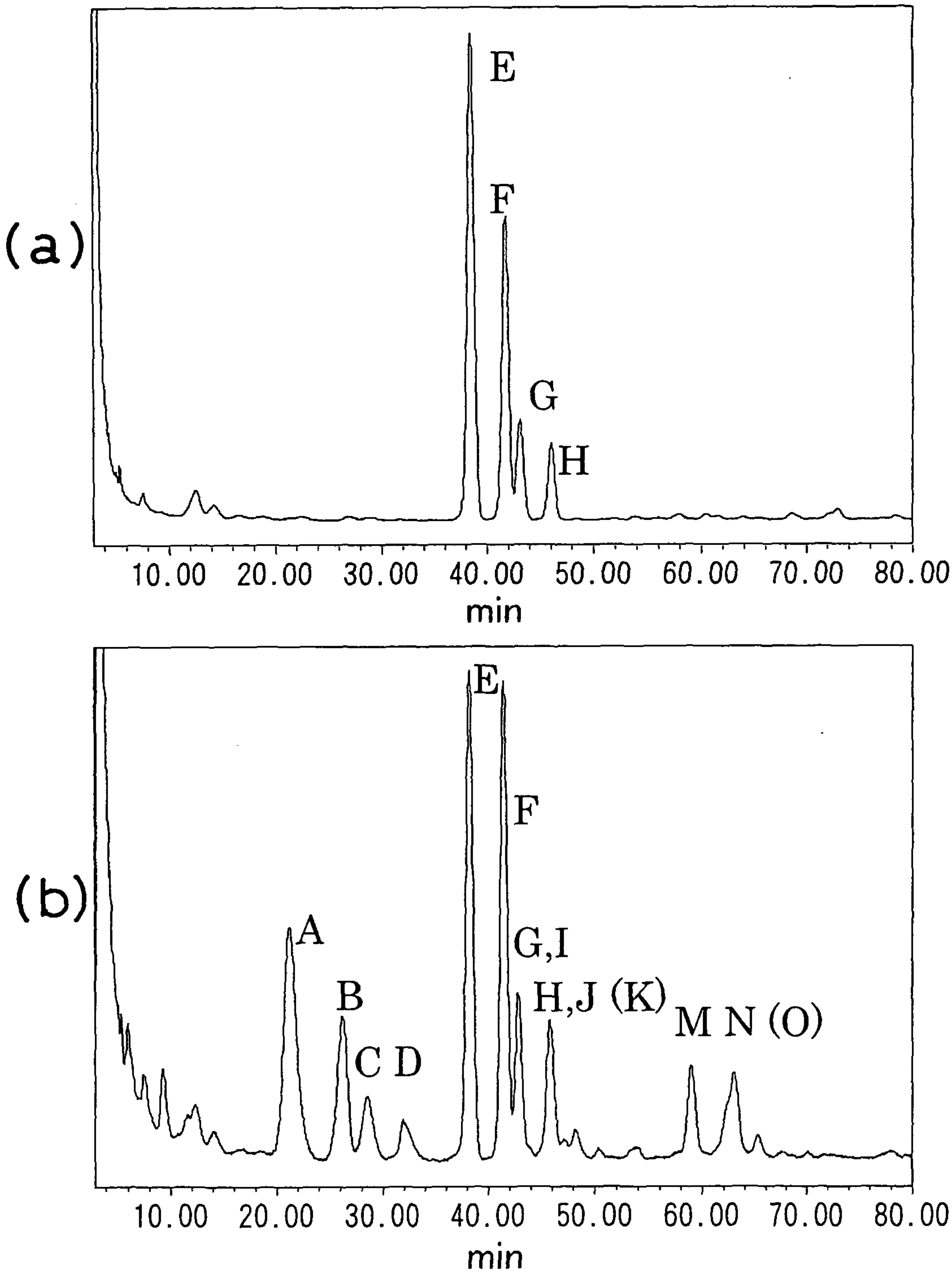
(a)
E
F
G
H
10.00 20.00 30.00 40.00 50.00 60.00 70.00 80.00
min
(b)
E
F
A
G,I
B
H,J (K)
M N (O)
C D
10.00 20.00 30.00 40.00 50.00 60.00 70.00 80.00
min

# Fig.5

**A**

GlcNAcβ1 - 2Manα1 \ 6
Manβ1-4GlcNAcβ1-4GlcNAc-PA
GlcNAcβ1 - 2Manα1 / 3

**B**

Galβ1 - 4GlcNAcβ1 - 2Manα1 \ 6
Manβ1-4GlcNAcβ1-4GlcNAc-PA
GlcNAcβ1 - 2Manα1 / 3

**C**

GlcNAcβ1 - 2Manα1 \ 6
Manβ1-4GlcNAcβ1-4GlcNAc-PA
Galβ1 - 4GlcNAcβ1 - 2Manα1 / 3

**D**

Galβ1 - 4GlcNAcβ1 - 2Manα1 \ 6
Manβ1-4GlcNAcβ1-4GlcNAc-PA
Galβ1 - 4GlcNAcβ1 - 2Manα1 / 3

**E**

GlcNAcβ1 - 2Manα1 \ 6
Fucα1 \ 6
Manβ1-4GlcNAcβ1-4GlcNAc-PA
GlcNAcβ1 - 2Manα1 / 3

**F**

Galβ1 - 4GlcNAcβ1 - 2Manα1 \ 6
Fucα1 \ 6
Manβ1-4GlcNAcβ1-4GlcNAc-PA
GlcNAcβ1 - 2Manα1 / 3

**G**

GlcNAcβ1 - 2Manα1 \ 6
Fucα1 \ 6
Manβ1-4GlcNAcβ1-4GlcNAc-PA
Galβ1 - 4GlcNAcβ1 - 2Manα1 / 3

**H**

Galβ1 - 4GlcNAcβ1 - 2Manα1 \ 6
Fucα1 \ 6
Manβ1-4GlcNAcβ1-4GlcNAc-PA
Galβ1 - 4GlcNAcβ1 - 2Manα1 / 3

# Fig.6

I
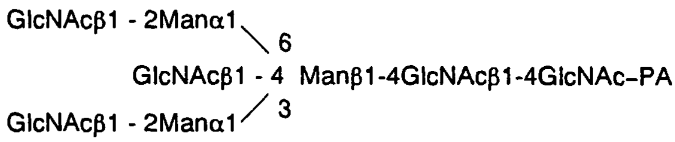

J
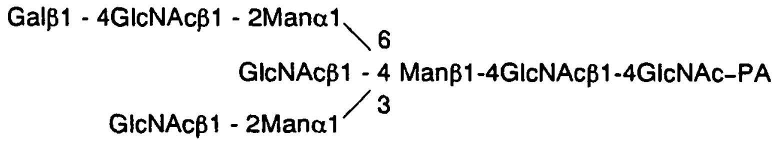

K
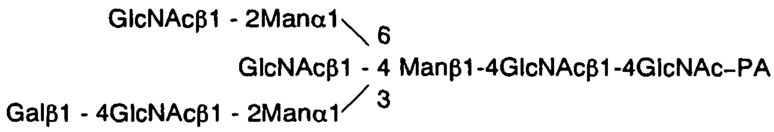

L
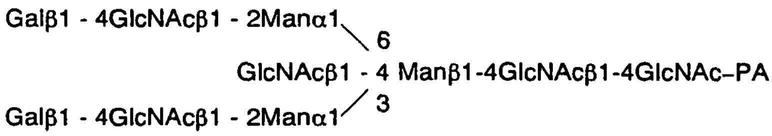

M
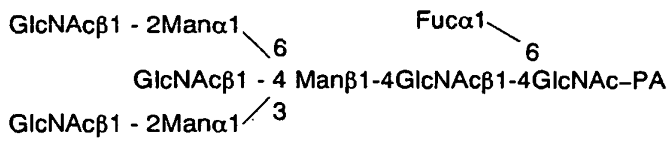

N
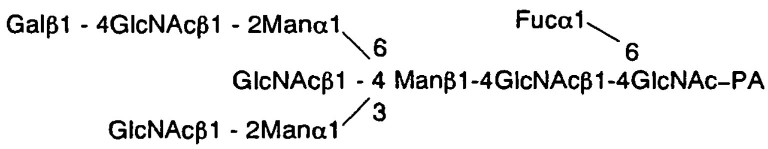

O
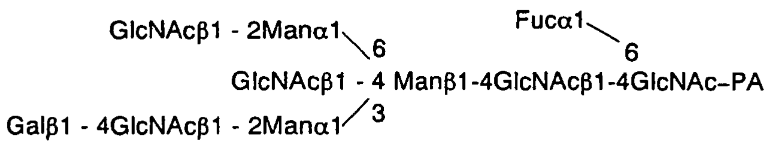

P
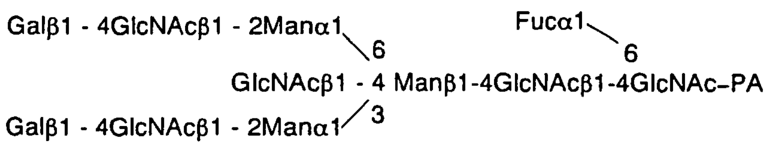

Fig.7

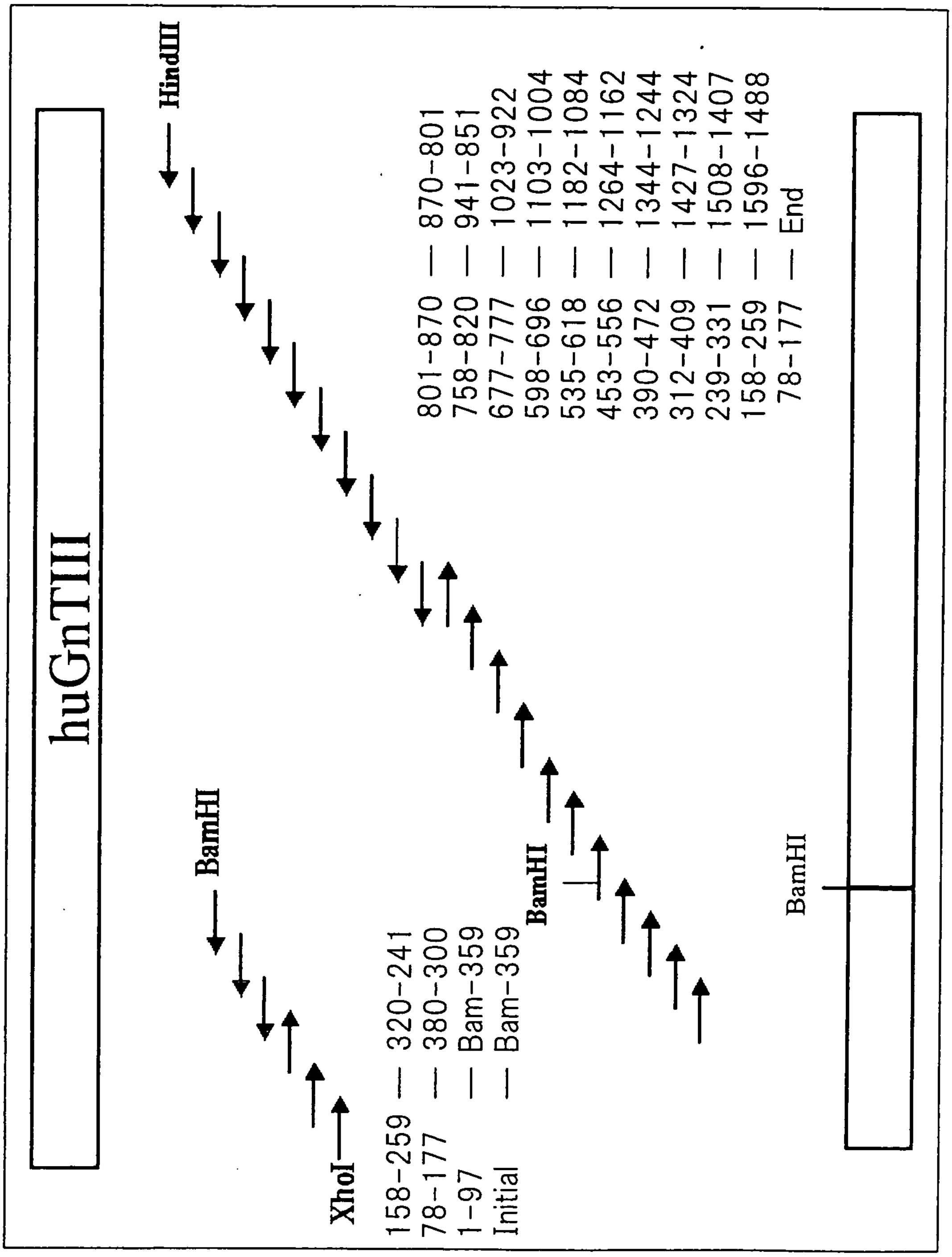
huGnTIII
HindIII
BamHI
XhoI
BamHI
BamHI
158-259 — 320-241
78-177 — 380-300
1-97 — Bam-359
Initial — Bam-359
801-870 — 870-801
758-820 — 941-851
677-777 — 1023-922
598-696 — 1103-1004
535-618 — 1182-1084
453-556 — 1264-1162
390-472 — 1344-1244
312-409 — 1427-1324
239-331 — 1508-1407
158-259 — 1596-1488
78-177 — End

Fig.8

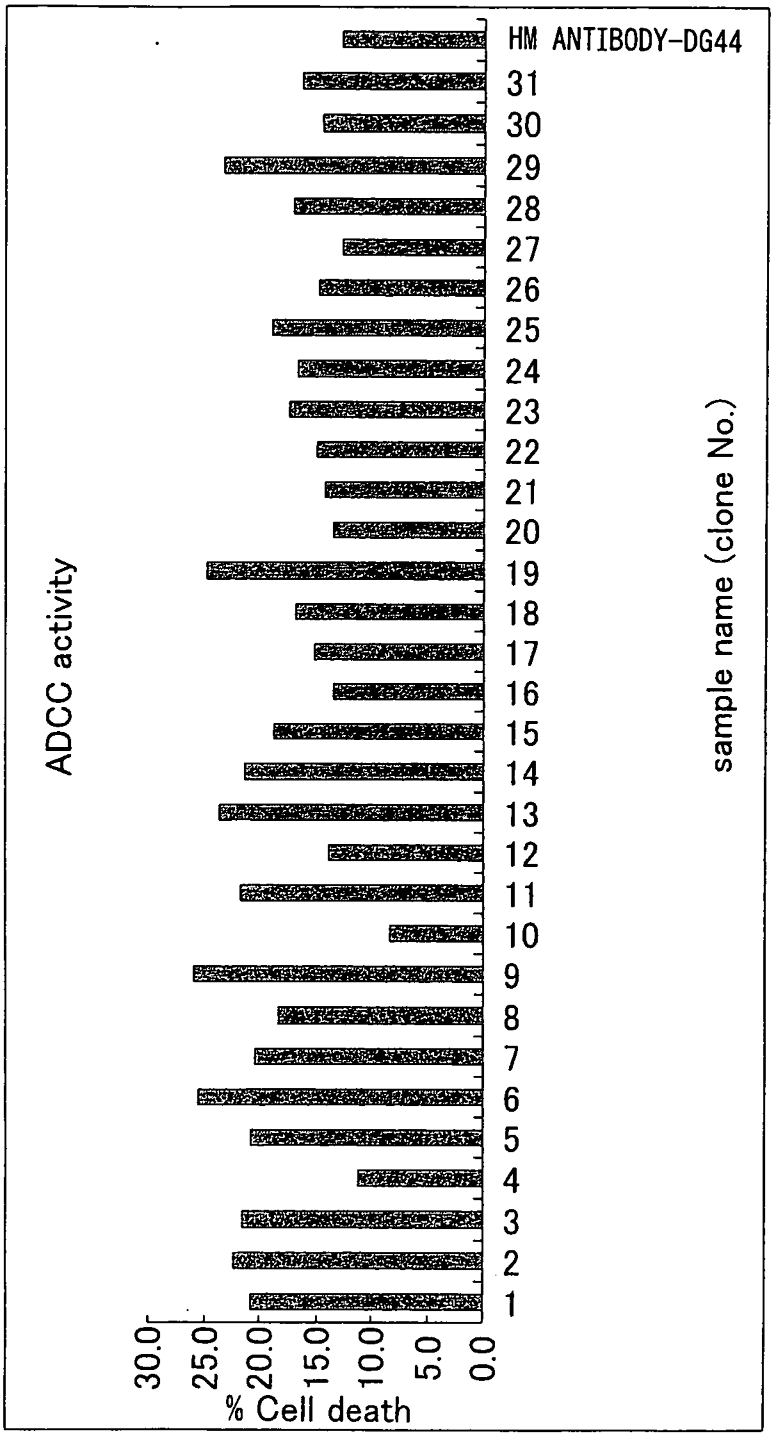
ADCC activity
% Cell death
30.0
25.0
20.0
15.0
10.0
5.0
0.0
1
2
3
4
5
6
7
8
9
10
11
12
13
14
15
16
17
18
19
20
21
22
23
24
25
26
27
28
29
30
31
HM ANTIBODY-DG44
sample name (clone No.)

Fig.9

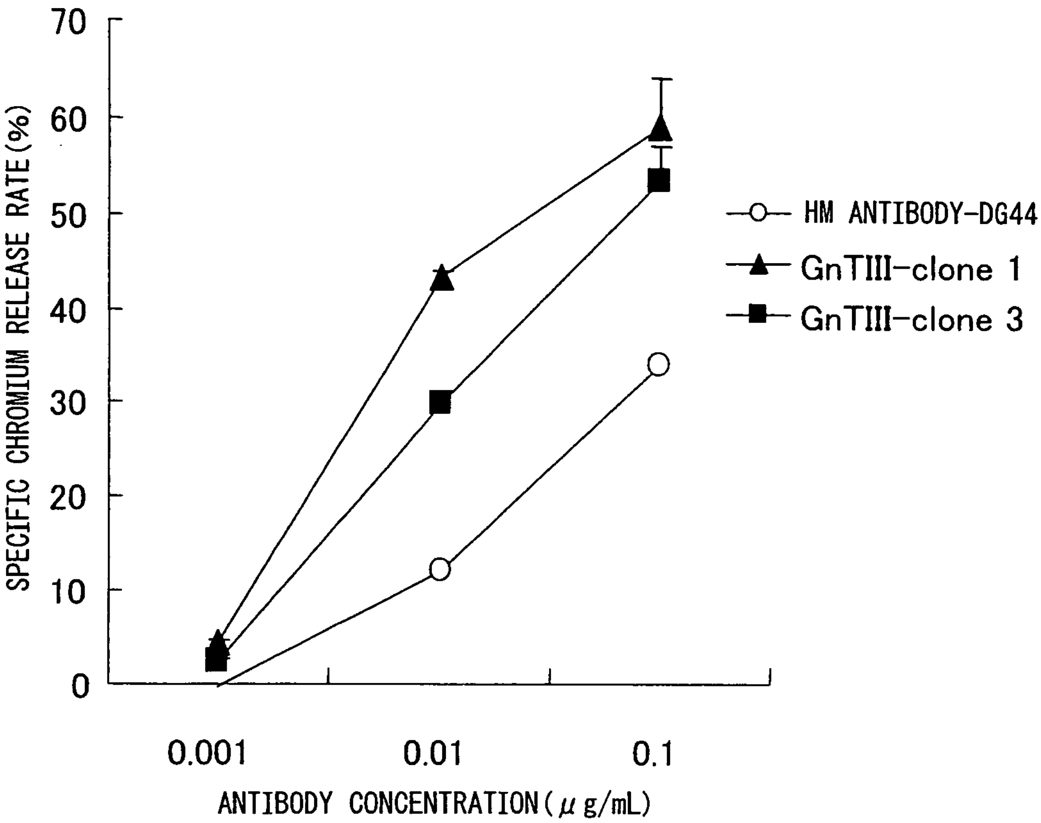
70
60
50
40
30
20
10
0
SPECIFIC CHROMIUM RELEASE RATE(%)
0.001
0.01
0.1
ANTIBODY CONCENTRATION(μg/mL)
HM ANTIBODY-DG44
GnTIII-clone 1
GnTIII-clone 3

# Fig.10

```
GnTIII mut.nuc    1:ATGAGACGCTACAAGCTCTTTCTCATGTTCTGTATGGCCGGCCTGTGCCTCATCTCCTTC 60
GnTIII ori.nuc    1:ATGAGACGCTACAAGCTCTTTCTCATGTTCTGTATGGCCGGCCTGTGCCTCATCTCCTTC 60
                    ************************************************************

GnTIII mt.nuc    61:CTGCACTTCTTCAAGACCCTGTCCTATGTCACCTTCCCACGAGAACTGGCCTCCCTCAGC 120
GnTIII ori.nuc   61:CTGCACTTCTTCAAGACCCTGTCCTATGTCACCTTCCCCCGAGAACTGGCCTCCCTCAGC 120
                    ************************************** *********************

GnTIII mt.nuc   121:CCTAACCTGGTGTCCAGCTTTTTCTGGAACAATGCCCCGGTCACGCCCCAGGCCAGCCCT 180
GnTIII ori.nuc  121:CCTAACCTGGTGTCCAGCTTTTTCTGGAACAATGCCCCGGTCACGCCCCAGGCCAGCCCC 180
                    ***********************************************************

GnTIII mt.nuc   181:GAGCCAGGAGGCCCTGACCTGCTGCGTACCCCACTCTACTCCCACTCGCCCCTGCTGCAG 240
GnTIII ori.nuc  181:GAGCCAGGAGGCCCTGACCTGCTGCGTACCCCACTCTACTCCCACTCGCCCCTGCTGCAG 240
                    ************************************************************

GnTIII mt.nuc   241:CCGCTGCCGCCCAGCAAGGCGGCCGAGGAGCTCCACCGGGTGGACTTGGTGCTGCCCGAG 300
GnTIII ori.nuc  241:CCGCTGCCGCCCAGCAAGGCGGCCGAGGAGCTCCACCGGGTGGACTTGGTGCTGCCCGAG 300
                    ************************************************************
```

# Fig.11

```
GnTIII mt.nuc   301:GACACCACCGAGTATTTCGTGCGCACCAAGGCTGGAGGCGTCTGCTTCAAACCCGGCACC 360
GnTIII ori.nuc  301:GACACCACCGAGTATTTCGTGCGCACCAAGGCCGGCGGCGTCTGCTTCAAACCCGGCACC 360
                    ******************************** ** ************************

GnTIII mt.nuc   361:AAGATGCTGGAGAGACCGCCTCCGGGACGACCGGAGGAGAAGCCTGAGGGGGCCAACGGA 420
GnTIII ori.nuc  361:AAGATGCTGGAGAGGCCGCCCCCGGGACGGCCGGAGGAGAAGCCTGAGGGGGCCAACGGC 420
                    ************** ***** ******** *****************************

GnTIII mt.nuc   421:TCCTCGGCCCGGCGACCACCCCGGTACCTCCTGAGCGCCCGGGAGCGCACGGGGGGCCGA 480
GnTIII ori.nuc  421:TCCTCGGCCCGGCGGCCACCCCGGTACCTCCTGAGCGCCCGGGAGCGCACGGGGGGCCGA 480
                    ************** *********************************************

GnTIII mt.nuc   481:GGTGCACGACGCAAGTGGGTGGAGTGCGTGTGTCTGCCCGGATGGCACGGACCCAGCTGC 540
GnTIII ori.nuc  481:GGCGCCCGGCGCAAGTGGGTGGAGTGCGTGTGCCTGCCCGGCTGGCACGGACCCAGCTGC 540
                    ** ** ** *********************** ******** ******************

GnTIII mt.nuc   541:GGCGTGCCCACTGTGGTGCAGTATTCCAACCTGCCTACCAAGGAGCGGCTGGTGCCCAGG 600
GnTIII ori.nuc  541:GGCGTGCCCACTGTGGTGCAGTACTCCAACCTGCCCACCAAGGAGCGGCTGGTGCCCAGG 600
                    *********************** *********** ************************
```

# Fig.12

```
GnTIII mt.nuc   601:GAGGTGCCGCGCCGCGTCATTAATGCTATCAACGTCAACCACGAGTTCGACCTGCTGGAC 660
GnTIII ori.nuc  601:GAGGTGCCGCGCCGCGTCATCAACGCCATCAACGTCAACCACGAGTTCGACCTGCTGGAC 660
                    ******************** ** ** *********************************

GnTIII mt.nuc   661:GTGCGCTTCCACGAGCTGGGCGACGTGGTGGACGCCTTTGTGGTGTGCGAGTCCAACTTC 720
GnTIII ori.nuc  661:GTGCGCTTCCACGAGCTGGGCGACGTGGTGGACGCCTTTGTGGTGTGCGAGTCCAACTTC 720
                    ************************************************************

GnTIII mt.nuc   721:ACGGCTTATGGGGAGCCGCGGCCGCTCAAGTTCCGGGAGATGCTGACCAATGGCACCTTC 780
GnTIII ori.nuc  721:ACGGCTTATGGGGAGCCGCGGCCGCTCAAGTTCCGGGAGATGCTGACCAATGGCACCTTC 780
                    ************************************************************

GnTIII mt.nuc   781:GAGTACATCCGCCACAAGGTGCTCTATGTCTTCCTGGACCACTTTCCTCCTGGAGGACGA 840
GnTIII ori.nuc  781:GAGTACATCCGCCACAAGGTGCTCTATGTCTTCCTGGACCACTTCCCGCCCGGCGGCCGG 840
                    ******************************************** ** ** ** ** ** 

GnTIII mt.nuc   841:CAAGATGGATGGATCGCCGACGACTACCTGCGCACCTTCCTCACCCAGGACGGCGTCTCG 900
GnTIII ori.nuc  841:CAGGACGGCTGGATCGCCGACGACTACCTGCGCACCTTCCTCACCCAGGACGGCGTCTCG 900
                    ** ** ** ***************************************************
```

# Fig.13

```
GnTIII mt.nuc    901:CGGCTGCGCAACCTGCGGCCCGACGACGTCTTCATCATTGACGATGCGGACGAGATCCCG 960
GnTIII ori.nuc   901:CGGCTGCGCAACCTGCGGCCCGACGACGTCTTCATCATTGACGATGCGGACGAGATCCCG 960
                     ************************************************************

GnTIII mt.nuc    961:GCCCGTGACGGCGTCCTGTTCCTCAAGCTCTACGATGGCTGGACCGAGCCCTTCGCCTTC 1020
GnTIII ori.nuc   961:GCCCGTGACGGCGTCCTTTTCCTCAAGCTCTACGATGGCTGGACCGAGCCCTTCGCCTTC 1020
                     ***************** ******************************************

GnTIII mt.nuc   1021:CACATGCGCACGTCGCTCTACGGATTCTTTTGGAAGCAACCGGGCACCCTGGAGGTGGTG 1080
GnTIII ori.nuc  1021:CACATGCGCACGTCGCTCTACGGCTTCTTCTGGAAGCAGCCGGGCACCCTGGAGGTGGTG 1080
                     *********************** ***** ******** *********************

GnTIII mt.nuc   1081:TCAGGCTGCACGGTGGACATGCTGCAGGCAGTGTATGGGCTGGACGGCATCCGCCTGCGC 1140
GnTIII ori.nuc  1081:TCAGGCTGCACGGTGGACATGCTGCAGGCAGTGTATGGGCTGGACGGCATCCGCCTGCGC 1140
                     ************************************************************

GnTIII mt.nuc   1141:CGCCGCCAATACTACACCATGCCCAACTTCAGACAGTATGAGAACCGCACCGGACACATC 1200
GnTIII ori.nuc  1141:CGCCGCCAGTACTACACCATGCCCAACTTCAGACAGTATGAGAACCGCACCGGCCACATC 1200
                     ******** ******************************************** ******
```

# Fig.14

```
GnTIII mt.nuc  1201:CTGGTGCAGTGGTCGCTGGGCAGCCCCCTTCACTTCGCCGGCTGGCACTGCTCCTGGTGC 1260
GnTIII ori.nuc 1201:CTGGTGCAGTGGTCGCTGGGCAGCCCCCTGCACTTCGCCGGCTGGCACTGCTCCTGGTGC 1260
                    ***************************** ******************************

GnTIII mt.nuc  1261:TTCACGCCCGAGGGCATCTACTTCAAGCTCGTGTCCGCCCAGAATGGCGACTTCCCACGC 1320
GnTIII ori.nuc 1261:TTCACGCCCGAGGGCATCTACTTCAAGCTCGTGTCCGCCCAGAATGGCGACTTCCCACGC 1320
                    ************************************************************

GnTIII mt.nuc  1321:TGGGGTGACTACGAGGACAAGCGGGACCTGAACTACATCCGCGGCCTGATCCGCACCGGG 1380
GnTIII ori.nuc 1321:TGGGGTGACTACGAGGACAAGCGGGACCTGAACTACATCCGCGGCCTGATCCGCACCGGG 1380
                    ************************************************************

GnTIII mt.nuc  1381:GGCTGGTTCGACGGCACGCAGCAAGAGTACCCGCCTGCAGACCCCAGCGAGCACATGTAT 1440
GnTIII ori.nuc 1381:GGCTGGTTCGACGGCACGCAGCAGGAGTACCCGCCTGCAGACCCCAGCGAGCACATGTAT 1440
                    *********************** ************************************

GnTIII mt.nuc  1441:GCGCCCAAGTACCTGCTGAAGAACTACGACCGGTTCCACTACCTTCTGGACAACCCCTAC 1500
GnTIII ori.nuc 1441:GCGCCCAAGTACCTGCTGAAGAACTACGACCGGTTCCACTACCTGCTGGACAACCCCTAC 1500
                    ******************************************* ****************
```

# Fig.15

```
GnTIII mt.nuc  1501:CAGGAGCCCAGGAGCACGGCTGCGGGAGGATGGCGCCACAGGGGTCCTGAAGGAAGACCG 1560
GnTIII ori.nuc 1501:CAGGAGCCCAGGAGCACGGCGGCGGGCGGGTGGCGCCACAGGGGTCCCGAGGGAAGGCCG 1560
                    ******************** ***** ** ***************** ** ***** ***

GnTIII mt.nuc  1561:CCTGCTCGGGGAAAACTGGACGAGGCGGAAGTCTAG                        1596
GnTIII ori.nuc 1561:CCCGCCCGGGGCAAACTGGACGAGGCGGAAGTCTAG                        1596
                    ** ** ***** ************************
```

**INTERNATIONAL SEARCH REPORT**

International application No. PCT/JP2004/011812

A. CLASSIFICATION OF SUBJECT MATTER
Int.Cl$^7$ C07K16/30, C12N15/12, C12P21/08

According to International Patent Classification (IPC) or to both national classification and IPC

B. FIELDS SEARCHED

Minimum documentation searched (classification system followed by classification symbols)
Int.Cl$^7$ C07K16/30, C12N15/12, C12P21/08

Documentation searched other than minimum documentation to the extent that such documents are included in the fields searched

Electronic data base consulted during the international search (name of data base and, where practicable, search terms used)
BIOSIS/WPI(DIALOG)

C. DOCUMENTS CONSIDERED TO BE RELEVANT

| Category* | Citation of document, with indication, where appropriate, of the relevant passages | Relevant to claim No. |
|---|---|---|
| Y | WO 98/37913 A1 (Chugai Pharmaceutical Co., Ltd.), 03 September, 1998 (03.09.98), Full text & JP 10-298106 A & EP 972524 A1 & US 2002/034507 A1 | 1-12 |
| Y | JP 10-286088 A (Chugai Pharmaceutical Co., Ltd.), 27 October, 1998 (27.10.98), Full text & WO 98/35698 A1 & EP 997152 A1 | 1-12 |
| Y | WO 02/31140 A1 (Kyowa Hakko Kogyo Co., Ltd.), 18 April, 2002 (18.04.02), Full text & EP 1331266 A1 | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 October, 2004 (20.10.04) | 09 November, 2004 (09.11.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No. PCT/JP2004/011812

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2002-512014 A (UNAMA, Pablo), 23 April, 2002 (23.04.02), Full text & WO 99/54342 A1 | 1-12 |
| Y | WO 02/79255 A1 (IDEC PHARMACEUTICALS Co.), 10 October, 2002 (10.10.02), Full text (Family: none) | 1-12 |

Form PCT/ISA/210 (continuation of second sheet) (January 2004)

**INTERNATIONAL SEARCH REPORT**

International application No.
PCT/JP2004/011812

**Box No. I Nucleotide and/or amino acid sequence(s) (Continuation of item1.b of the first sheet)**

1. With regard to any nucleotide and/or amino acid sequence disclosed in the international application and necessary to the claimed invention, the international search was carried out on the basis of:

   a. type of material
      - [x] a sequence listing
      - [ ] table(s) related to the sequence listing

   b. format of material
      - [ ] in written format
      - [x] in computer readable form

   c. time of filing/furnishing
      - [ ] contained in the international application as filed
      - [x] filed together with the international application in computer readable form
      - [ ] furnished subsequently to this Authority for the purposes of search

2. [x] In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (January 2004)

SEQUENCE LISTING

<110>Chugai Seiyaku Kabushiki Kaisha

<120>Anti HM1.24 Antibody With Modified Sugar Chains

<130>P817

<150>JP 2003-207165

<151>2003-08-11

<160>29

<210>1

<211>39

<221>DNA

<213>Artificial Sequence

<220>

<221>

<222>

<223>Foward primer Initial(BamHI)

<400>1

tttctcgaga tgagacgcta caagctcttt ctcatgttc 39

<210>2

<211>97

<221>DNA

<213>Artificial Sequence

<220>

<221>

<222>

<223>Foward primer 1-97

<400>2

atgagacgct acaagctctt tctcatgttc tgtatggccg gcctgtgcct catctccttc 60

ctgcacttct tcaagaccct gtcctatgtc accttcc 97

<210>3

<211>100

<221>DNA

<213>Artificial Sequence

<220>

<221>

<222>

<223>Foward primer 78-177

<400>3
cctgtcctat gtcaccttcc cacgagaact ggcctccctc agccctaacc tggtgtccag 60
ctttttctgg aacaatgccc cggtcacgcc ccaggccagc 100
<210>4
<211>102
<221>DNA
<213>Artificial Sequence
<220>
<221>
<222>
<223>Foward primer 158-259
<400>4
cggtcacgcc ccaggccagc cctgagccag gaggccctga cctgctgcgt accccactct 60
actcccactc gcccctgctg cagccgctgc cgcccagcaa gg 102
<210>5
<211>93
<221>DNA
<213>Artificial Sequence
<220>
<221>
<222>
<223>Foward primer 239-331
<400>5
agccgctgcc gcccagcaag gcggccgagg agctccaccg ggtggacttg gtgctgcccg 60
aggacaccac cgagtatttc gtgcgcacca agg 93
<210>6
<211>98
<221>DNA
<213>Artificial Sequence
<220>
<221>
<222>
<223>Foward primer 312-409
<400>6
gtatttcgtg cgcaccaagg ctggaggcgt ctgcttcaaa cccggcacca agatgctgga 60
gagaccgcct ccgggacgac cggaggagaa gcctgagg 98

<210>7
<211>83
<221>DNA
<213>Artificial Sequence
<220>
<221>
<222>
<223>Foward primer 390-472
<400>7
accggaggag aagcctgagg gggccaacgg atcctcggcc cggcgaccac cccggtacct 60
cctgagcgcc cgggagcgca cgg 83
<210>8
<211>104
<221>DNA
<213>Artificial Sequence
<220>
<221>
<222>
<223>Foward primer 453-556
<400>8
gagcgcccgg gagcgcacgg ggggccgagg tgcacgacgc aagtgggtgg agtgcgtgtg 60
tctgcccgga tggcacggac ccagctgcgg cgtgcccact gtgg 104
<210>9
<211>84
<221>DNA
<213>Artificial Sequence
<220>
<221>
<222>
<223>Foward primer 535-618
<400>9
agctgcggcg tgcccactgt ggtgcagtat tccaacctgc ctaccaagga gcggctggtg 60
cccagggagg tgccgcgccg cgtc 84
<210>10
<211>99
<221>DNA

<213>Artificial Sequence

<220>

<221>

<222>

<223>Foward primer 598-696

<400>10

agggaggtgc cgcgccgcgt cattaatgct atcaacgtca accacgagtt cgacctgctg 60

gacgtgcgct tccacgagct gggcgacgtg gtggacgcc 99

<210>11

<211>101

<221>DNA

<213>Artificial Sequence

<220>

<221>

<222>

<223>Foward primer 677-777

<400>11

tgggcgacgt ggtggacgcc tttgtggtgt gcgagtccaa cttcacggct tatggggagc 60

cgcggccgct caagttccgg gagatgctga ccaatggcac c 101

<210>12

<211>63

<221>DNA

<213>Artificial Sequence

<220>

<221>

<222>

<223>Foward primer 758-820

<400>12

agatgctgac caatggcacc ttcgagtaca tccgccacaa ggtgctctat gtcttcctgg 60

acc 63

<210>13

<211>70

<221>DNA

<213>Artificial Sequence

<220>

<221>

<222>

<223>Foward primer 801-870

<400>13

gctctatgtc ttcctggacc actttcctcc tggaggacga caagatggat ggatcgccga 60

cgactacctg 70

<210>14

<211>37

<221>DNA

<213>Artificial Sequence

<220>

<221>

<222>

<223>Reverse primer End(HindIII)

<400>14

tttaagctta ctagacttcc gcctcgtcca gttttcc 37

<210>15

<211>109

<221>DNA

<213>Artificial Sequence

<220>

<221>

<222>

<223>Reverse primer 1596-1488

<400>15

ctagacttcc gcctcgtcca gttttccccg agcaggcggt cttccttcag gacccctgtg 60

gcgccatcct cccgcagccg tgctcctggg ctcctggtag gggttgtcc 109

<210>16

<211>102

<221>DNA

<213>Artificial Sequence

<220>

<221>

<222>

<223>Reverse primer 1508-1407

<400>16

ggctcctggt aggggttgtc cagaaggtag tggaaccggt cgtagttctt cagcaggtac 60

ttgggcgcat acatgtgctc gctggggtct gcaggcgggt ac 102

<210>17

<211>104

<221>DNA

<213>Artificial Sequence

<220>

<221>

<222>

<223>Reverse primer 1427-1324

<400>17

ctggggtctg caggcgggta ctcttgctgc gtgccgtcga accagccccc ggtgcggatc 60

aggccgcgga tgtagttcag gtcccgcttg tcctcgtagt cacc 104

<210>18

<211>101

<221>DNA

<213>Artificial Sequence

<220>

<221>

<222>

<223>Reverse primer 1344-1244

<400>18

ccgcttgtcc tcgtagtcac cccagcgtgg gaagtcgcca ttctgggcgg acacgagctt 60

gaagtagatg ccctcgggcg tgaagcacca ggagcagtgc c 101

<210>19

<211>102

<221>DNA

<213>Artificial Sequence

<220>

<221>

<222>

<223>Reverse primer 1264-1162

<400>19

tgaagcacca ggagcagtgc cagccggcga agtgaagggg gctgcccagc gaccactgca 60

ccaggatgtg tccggtgcgg ttctcatact gtctgaagtt gg 102

<210>20

<211>99

<221>DNA
<213>Artificial Sequence
<220>
<221>
<222>
<223>Reverse primer 1182-1084
<400>20
ctcatactgt ctgaagttgg gcatggtgta gtattggcgg cggcgcaggc ggatgccgtc 60
cagcccatac actgcctgca gcatgtccac cgtgcagcc 99
<210>21
<211>100
<221>DNA
<213>Artificial Sequence
<220>
<221>
<222>
<223>Reverse primer 1103-1004
<400>21
agcatgtcca ccgtgcagcc tgacaccacc tccagggtgc ccggttgctt ccaaaagaat 60
ccgtagagcg acgtgcgcat gtggaaggcg aagggctcgg 100
<210>22
<211>102
<221>DNA
<213>Artificial Sequence
<220>
<221>
<222>
<223>Reverse primer 1023-922
<400>22
gtggaaggcg aagggctcgg tccagccatc gtagagcttg aggaacagga cgccgtcacg 60
ggccgggatc tcgtccgcat cgtcaatgat gaagacgtcg tc 102
<210>23
<211>91
<221>DNA
<213>Artificial Sequence
<220>

<221>
<222>
<223>Reverse primer 941-851
<400>23
tcaatgatga agacgtcgtc gggccgcagg ttgcgcagcc gcgagacgcc gtcctgggtg 60
aggaaggtgc gcaggtagtc gtcggcgatc c 91
<210>24
<211>70
<221>DNA
<213>Artificial Sequence
<220>
<221>
<222>
<223>Reverse primer 870-801
<400>24
caggtagtcg tcggcgatcc atccatcttg tcgtcctcca ggaggaaagt ggtccaggaa 60
gacatagagc 70
<210>25
<211>81
<221>DNA
<213>Artificial Sequence
<220>
<221>
<222>
<223>Reverse primer 380-300
<400>25
ggcggtctct ccagcatctt ggtgccgggt ttgaagcaga cgcctccagc cttggtgcgc 60
acgaaatact cggtggtgtc c 81
<210>26
<211>80
<221>DNA
<213>Artificial Sequence
<220>
<221>
<222>
<223>Reverse primer 320-241

<400>26

acgaaatact cggtggtgtc ctcgggcagc accaagtcca cccggtggag ctcctcggcc 60

gccttgctgg gcggcagcgg 80

<210>27

<211>68

<221>DNA

<213>Artificial Sequence

<220>

<221>

<222>

<223>Reverse primer Bam-359

<400>27

tttggatccg ttggccccct caggcttctc ctccggtcgt cccggaggcg gtctctccag 60

catcttgg 68

<210>28

<211>1596

<221>DNA

<213>Homo sapiens

<220>

<223>Nucleotide sequence encoding human GnTIII

<400>28

atgagacgct acaagctctt tctcatgttc tgtatggccg gcctgtgcct catctccttc 60

ctgcacttct tcaagaccct gtcctatgtc accttccccc gagaactggc ctccctcagc 120

cctaacctgg tgtccagctt tttctggaac aatgccccgg tcacgcccca ggccagcccc 180

gagccaggag gccctgacct gctgcgtacc ccactctact cccactcgcc cctgctgcag 240

ccgctgccgc ccagcaaggc ggccgaggag ctccaccggg tggacttggt gctgcccgag 300

gacaccaccg agtatttcgt gcgcaccaag gccggcggcg tctgcttcaa acccggcacc 360

aagatgctgg agaggccgcc cccgggacgg ccggaggaga agcctgaggg ggccaacggc 420

tcctcggccc ggcggccacc ccggtacctc ctgagcgccc gggagcgcac gggggggccga 480

ggcgcccggc gcaagtgggt ggagtgcgtg tgcctgcccg gctggcacgg acccagctgc 540

ggcgtgccca ctgtggtgca gtactccaac ctgcccacca aggagcggct ggtgcccagg 600

gaggtgccgc gccgcgtcat caacgccatc aacgtcaacc acgagttcga cctgctggac 660

gtgcgcttcc acgagctggg cgacgtggtg gacgcctttg tggtgtgcga gtccaacttc 720

acggcttatg gggagccgcg gccgctcaag ttccgggaga tgctgaccaa tggcaccttc 780

gagtacatcc gccacaaggt gctctatgtc ttcctggacc acttcccgcc cggcggccgg 840

caggacggct ggatcgccga cgactacctg cgcaccttcc tcacccagga cggcgtctcg 900

cggctgcgca acctgcggcc cgacgacgtc ttcatcattg acgatgcgga cgagatcccg 960
gcccgtgacg gcgtcctttt cctcaagctc tacgatggct ggaccgagcc cttcgccttc 1020
cacatgcgca cgtcgctcta cggcttcttc tggaagcagc cgggcaccct ggaggtggtg 1080
tcaggctgca cggtggacat gctgcaggca gtgtatgggc tggacggcat ccgcctgcgc 1140
cgccgccagt actacaccat gcccaacttc agacagtatg agaaccgcac cggccacatc 1200
ctggtgcagt ggtcgctggg cagcccctg cacttcgccg gctggcactg ctcctggtgc 1260
ttcacgcccg agggcatcta cttcaagctc gtgtccgccc agaatggcga cttcccacgc 1320
tggggtgact acgaggacaa gcgggacctg aactacatcc gcggcctgat ccgcaccggg 1380
ggctggttcg acggcacgca gcaggagtac ccgcctgcag accccagcga gcacatgtat 1440
gcgcccaagt acctgctgaa gaactacgac cggttccact acctgctgga caacccctac 1500
caggagccca ggagcacggc ggcgggcggg tggcgccaca ggggtcccga gggaaggccg 1560
cccgcccggg gcaaactgga cgaggcggaa gtctag 1596

<210>29
<211>1596
<221>DNA
<213>Artificial Sequence
<220>
<221>
<222>
<223>Artificial nucleotide sequence encoding human GnTIII
<400>29

atgagacgct acaagctctt tctcatgttc tgtatggccg gcctgtgcct catctccttc 60
ctgcacttct tcaagaccct gtcctatgtc accttcccac gagaactggc ctccctcagc 120
cctaacctgg tgtccagctt tttctggaac aatgccccgg tcacgcccca ggccagccct 180
gagccaggag gccctgacct gctgcgtacc ccactctact cccactcgcc cctgctgcag 240
ccgctgccgc ccagcaaggc ggccgaggag ctccaccggg tggacttggt gctgcccgag 300
gacaccaccg agtatttcgt gcgcaccaag gctggaggcg tctgcttcaa acccggcacc 360
aagatgctgg agagaccgcc tccgggacga ccggaggaga agcctgaggg ggccaacgga 420
tcctcggccc ggcgaccacc ccggtacctc ctgagcgccc gggagcgcac gggggggccga 480
ggtgcacgac gcaagtgggt ggagtgcgtg tgtctgcccg gatggcacgg acccagctgc 540
ggcgtgccca ctgtggtgca gtattccaac ctgcctacca aggagcggct ggtgcccagg 600
gaggtgccgc gccgcgtcat taatgctatc aacgtcaacc acgagttcga cctgctggac 660
gtgcgcttcc acgagctggg cgacgtggtg gacgcctttg tggtgtgcga gtccaacttc 720
acggcttatg gggagccgcg gccgctcaag ttccgggaga tgctgaccaa tggcaccttc 780
gagtacatcc gccacaaggt gctctatgtc ttcctggacc actttcctcc tggaggacga 840
caagatggat ggatcgccga cgactacctg cgcaccttcc tcacccagga cggcgtctcg 900

```
cggctgcgca acctgcggcc cgacgacgtc ttcatcattg acgatgcgga cgagatcccg    960
gcccgtgacg gcgtcctgtt cctcaagctc tacgatggct ggaccgagcc cttcgccttc   1020
cacatgcgca cgtcgctcta cggattcttt tggaagcaac cgggcaccct ggaggtggtg   1080
tcaggctgca cggtggacat gctgcaggca gtgtatgggc tggacggcat ccgcctgcgc   1140
cgccgccaat actacaccat gcccaacttc agacagtatg agaaccgcac cggacacatc   1200
ctggtgcagt ggtcgctggg cagccccctt cacttcgccg gctggcactg ctcctggtgc   1260
ttcacgcccg agggcatcta cttcaagctc gtgtccgccc agaatggcga cttcccacgc   1320
tggggtgact acgaggacaa gcgggacctg aactacatcc gcggcctgat ccgcaccggg   1380
ggctggttcg acggcacgca gcaagagtac ccgcctgcag accccagcga gcacatgtat   1440
gcgcccaagt acctgctgaa gaactacgac cggttccact accttctgga caacccctac   1500
caggagccca ggagcacggc tgcgggagga tggcgccaca ggggtcctga aggaagaccg   1560
cctgctcggg gaaaactgga cgaggcggaa gtctag                             1596
```